# EUROPEAN PATENT APPLICATION

(11) **EP 3 300 668 A1**
(43) Date of publication of application: **04.04.2018**
(21) Application number: 16002303.2
(22) Date of filing: 28.10.2016
(51) Int. Cl.: A61B 10/02

(54) **DEVICE FOR BIOPSY WITH REUSABLE TRIGGER FOR DISPOSABLE NEEDLE**

(30) Priority: 30.09.2016 BR 102016022816
(71) Applicant: Paronetto, Dorival, Estado de Sao Paulo (BR)
(72) Inventor: Paronetto, Dorival, Estado de Sao Paulo (BR)
(74) Representative: Polo Flores, Luis Miguel

(57) **Abstract**

It consists of equipment for histological biopsy, characterized as medical equipment; composed of a set of disposable needles of the type "FULL CORE" and a reusable trigger for biopsy of soft tissues; the needles have been designed and developed for exclusive use in such trigger, and it is not possible to use them in other equipment; the inconvenient issues of the models of equipment for biopsy of the prior art are the user's discomfort and difficulty to handle them during the procedure; in most existing models, the user sets the equipment by making two movements to prepare it for triggering; after collection, it is necessary to set once to remove the sample and second time to remain in position for new collection; another inconvenient issue is related to the quantity of sample taken from the organ, which, in many cases, is not satisfactory to define the diagnosis; in order to solve these inconvenient issues, the object of the present patent petition was developed, whose principle of operation enables collection of the type "FULL CORE" (full cannula), in which the needle is moved by front (21) and back (22) carriers, which transport internal (7a) and external (6a) cannulas, providing their rotation movement, with mandrel (9a) remaining static; the carriers are moved by the traction of springs (41) placed in the front part of structural box (11); the trigger is also provided with a mechanism, which enables forward adjustment, easy to handle, to collect two sizes of samples with the same type of needle.

## Description

### TECHNICAL FIELD OF THE INVENTION

The purpose of this patent of right of invention is to present a practical and innovating model of device, composed of a reusable trigger for disposable needles of the type "FULL CORE" for soft tissue biopsy; the needles have been designed and developed for exclusive use in this trigger, and it is not possible to use them in other equipment; such device belongs to the field of medical equipment for specific use to take samples of tissues (human and animal) for the performance of their histological analysis and to which an original and innovating construction and utility disposition has been provided.

Thus, in the petition for the patent in question, we have an extremmely innovating equipment, especially designed and developed to obtain higher practicality and efficiency when used, bringing great advantages to its users. Simplicity of its conception to what refers to its manufacturing process can also be added.

The purpose of the present petition is also to present a new model of equipment for soft tissue biopsy with low industrialization costs; however, allied to the requirements for robustness, durability, safety and utility practicality, offering a fully innovating and high quality product for biopsy procedures to its users with quite competitive market value.

### BACKGROUND OF THE INVENTION

Biopsy is a surgical procedure, where the collection of tissue to be analyzed shall be made by medicine professionals from different specialties. Basically, there are two types of biopsy: cytological and histological.

Cytological biopsy is made through an aspiration puncture, using a small-diameter needle. It is an incision procedure, generally guided by ultrasonography, to aspire cysts or liquid content from a node or a tumor.

Histological biopsy consists in a percutaneous procedure carried out by means of a coaxial needle system, in which a small sample of tissue, where the lesion is located, is taken.

### DESCRIPTION OF PRIOR ARTS

Some equipment units used by now to perform histological biopsy are constituted of two carriers, with two linear movements in series and non-simultaneous, in a rectangular prismatic structure. In the front carrier, there is a cannula coupled and in the back carrier, there is a mandrel coupled with a recess close to its distal end. When the needle trigger is done, initially the mandrel dislocates penetrating in the region where the tissue sample will be taken for biopsy. This tissue settles in the recess of such mandrel and then, after the penetration of the mandrel, the cannula of the front carrier dislocates, cutting the tissue settled in the mandrel and collecting the material, considering that the movement of each carrier is provoked by independent helical springs. The principle of operation of such existing equipment is based on the spring compression.

One of the inconvenient issues related to the already existing equipment for biopsy is specifically the user's discomfort and difficulty to handle it during the procedure to make biopsy; furthermore, in most models, the user shall make two movements for the equipment to be ready to trigger.

Another inconvenient issue is after the collection of the tissue sample, where it is necessary to prepare once to remove it and a second time to place it in the correct position to collect again, in some procedures, it is necessary to collect several times, thus, causing big discomfort to the user.

Another inconvenient issue is related to the number of tissue samples taken from the organ, which, in many cases, are not satisfactory to make biopsy and define the diagnosis.

Thus, the disposition claimed herein seeks solving these inconvenient issues present in the prior art.

### BRIEF DESCRIPTION OF THE INVENTION

Considering the problems mentioned in the description of the prior art, it is clear that, by the present moment, there is still a need to develop equipment to make improvement in the process of taking samples for biopsy feasible.

After numberless searches and tests, the inventor, a person related to the area with recognized knowledge about the sector, created and developed the object of the present patent petition titled **"DISPOSITION APPLIED TO A DEVICE FOR BIOPSY WITH REUSABLE TRIGGER FOR DISPOSABLE NEEDLE",** which consists of equipment constituted of a set of needles and a trigger for biopsy, considering that the needles are for exclusive use of such trigger.

In this design, not only the mechanical and the functional qualities have been considered, but also the shape, the disposition and the localization of its parts and components, which, as they have been correctly placed, leaded to increase of efficiency, practicality and comfort to the procedure.

Thus, the present patent petition has been created and designed aiming at obtaining equipment with only one setting movement, conveniently configured for the performance of the biopsy procedure to allow the user to set it only once for each incision.

It is set for the first incision, the fragment of tissue is taken and at the same time, while it is being set for a new collection, the sample taken in the previous incision is removed.

Thus, a practical, efficient and innovating model of equipment of disposable needle and reusable trigger is presented, with all esthetical and functional qualities, designed and developed according to the modern engineering techniques.

This equipment was created to be highly durable, with practical operation and completely different from the models currently presented on the market, in ergonomic shape, which enables excellent level of functionality for the biopsy procedures.

The configuration of this biopsy equipment in its new construction shape is provided with a collection system with the purpose to promote easier penetration of the needle and collection of significantly higher quantity of tissue, making a coaxial rotation movement to the lumen.

The main advantages of such equipment are:
- Possibility to take bigger quantity of tissue fragments, making the diagnosis analysis more efficient;
- Set the equipment in an easier and more comfortable way, with a single and soft movement;
- Deep penetration for tissue collection, adjustable at 18 and 26 mm, with automatic return;
- Automatic opening of the lid to place the needle;
- Facility to place and remove the needle from the trigger, with "Poka-Yoke" system;
- Equipment with side or back and front triggers;
- Handling of the trigger with one hand only;
- The trigger can be sterilized in an autoclave (saturated water vapor);
- On its turn, the needle is a completely disposable device for single and exclusive use, provided with a system for longitudinal displacement of the set of cannulas (internal and external) and their simultaneous rotation movement, considering that such rotation movement occurs first at the beginning of the forward movement of the cannulas, cutting the tissue with the purpose to facilitate their penetration. After a short initial penetration course of the cannulas, this rotation

movement ends. After full penetration of the cannulas in the tissue to be taken, this rotation movement restarts with the purpose to cut off the tissue sample collected in the internal cannula, thus, avoiding excessive bleeding in the patient's organ.

It is thus comprehended that the equipment in question is extremely simple for manufacturing, associated to the practical and functional results obtained with its complete innovation over the equipment presented on the market.

### DESCRIPTION OF THE FIGURES

To complement the present description, in order to obtain higher comprehension of the characteristics of the present invention and according to its preferred practical performance, there is a set of drawings attached, where its operation is represented, but limited to, as an example, where it can be seen:

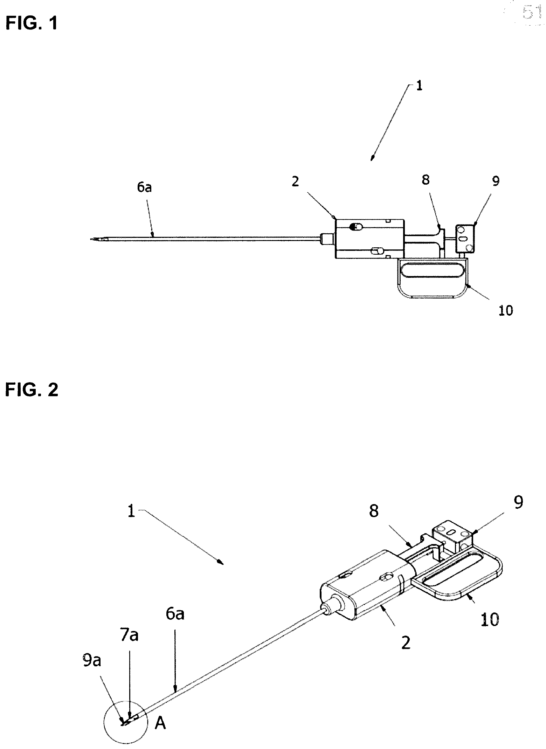

Figure 1 shows an upper view of the needle set with rotation system;
Figure 2 reveals an upper-front view of the needle set with rotation system;

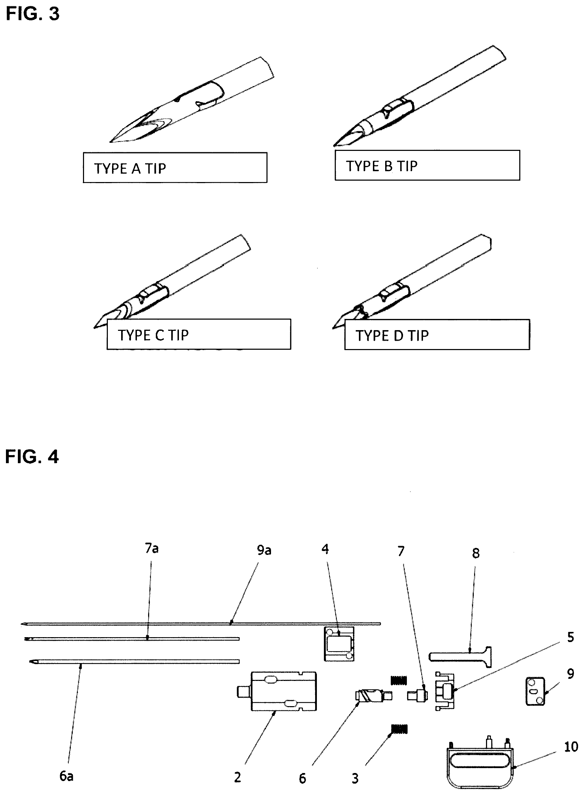

Figure 3 illustrates four upper-front views of the types of tips or bevels, which can be made on the needles;
Figure 4 shows an exploded upper view of the needle set with rotation system;

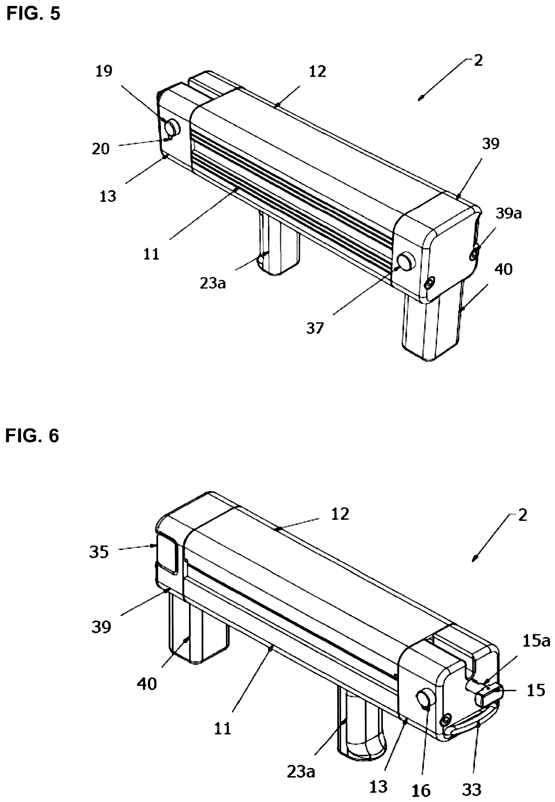

Figure 5 reveals a perspective upper-back view of the trigger, with a handle of the catch type, to be used with the needle with rotation system;
Figure 6 illustrates a perspective upper-front view of the trigger with a handle of the catch type;

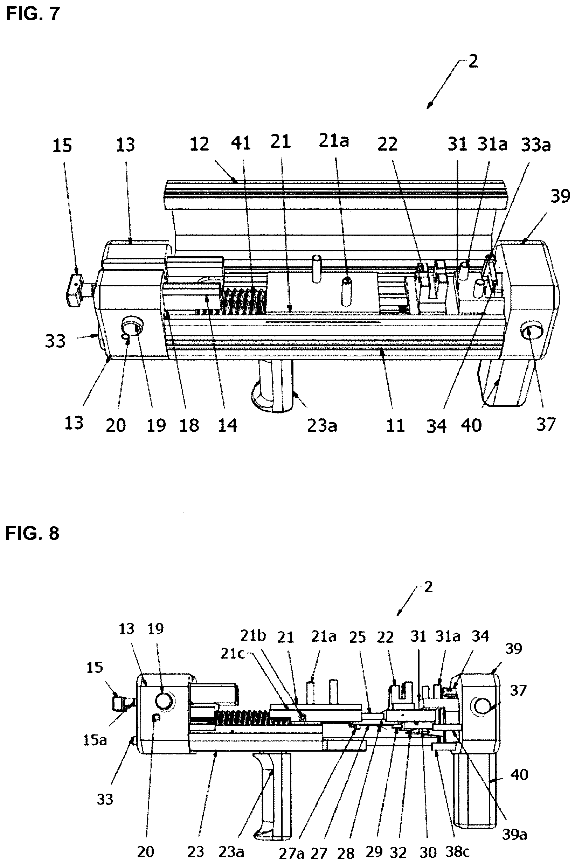

Figure 7 shows an upper-side view of the trigger without the structure, where the internal components can be seen;
Figure 8 reveals the side view of the trigger without the structure, where the internal components can be seen;

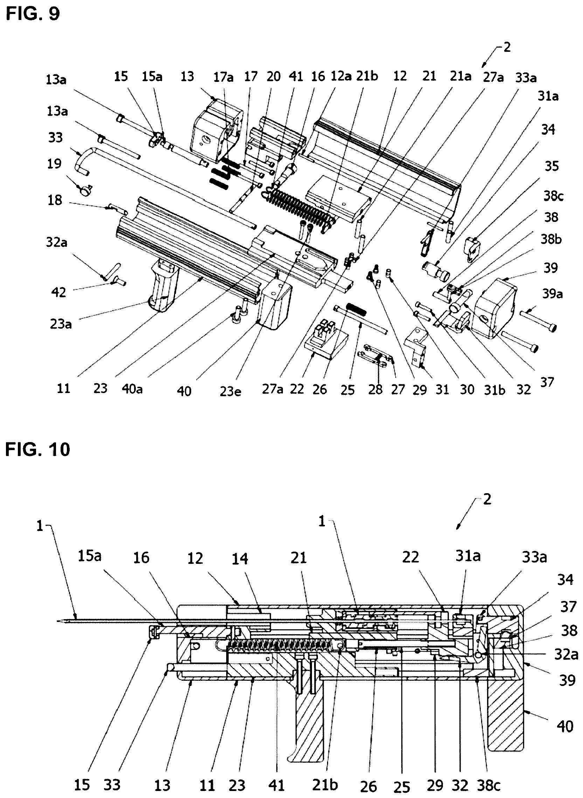

Figure 9 illustrates an exploded upper-back view, demonstrating the components of the trigger for biopsy needle with rotation system;
Figure 10 shows a side view of the trigger in full section, with the needle set coupled at set position;

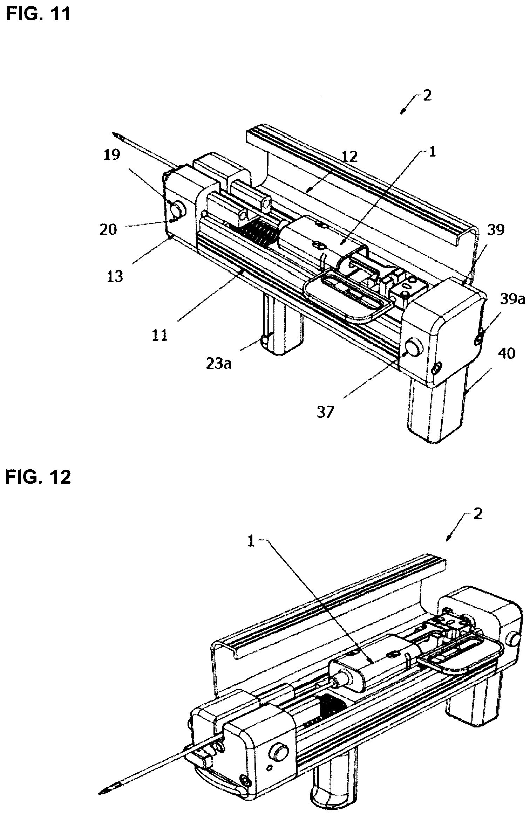

Figure 11 reveals an upper-back view of the trigger with the upper lid open and the needle set coupled at set position;
Figure 12 illustrates an upper-front view of the trigger with the lid and with the needle set coupled at set position;

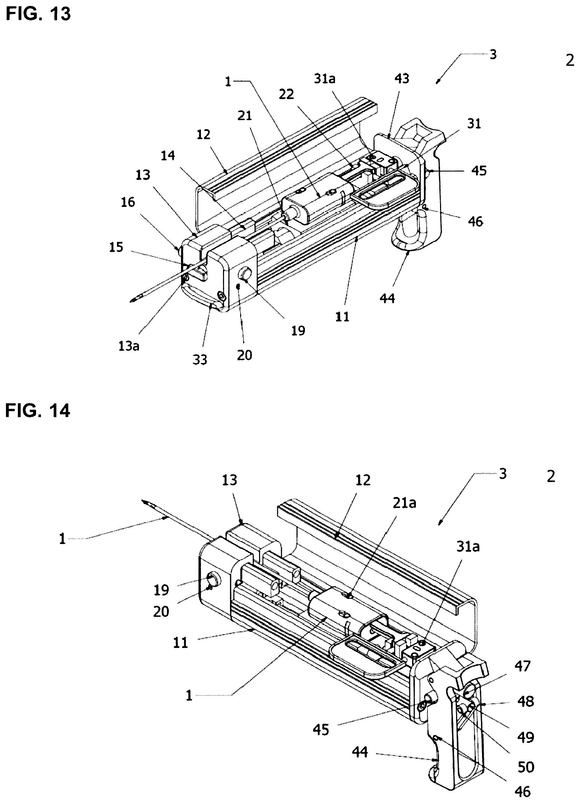

Figure 13 shows a perspective upper-front view of the trigger with the upper lid open and needle coupled, of the option of model with handle of the lever type;
Figure 14 reveals a perspective upper-back view of the trigger with the upper lid open and needle coupled, of the option of model with handle of the lever type;

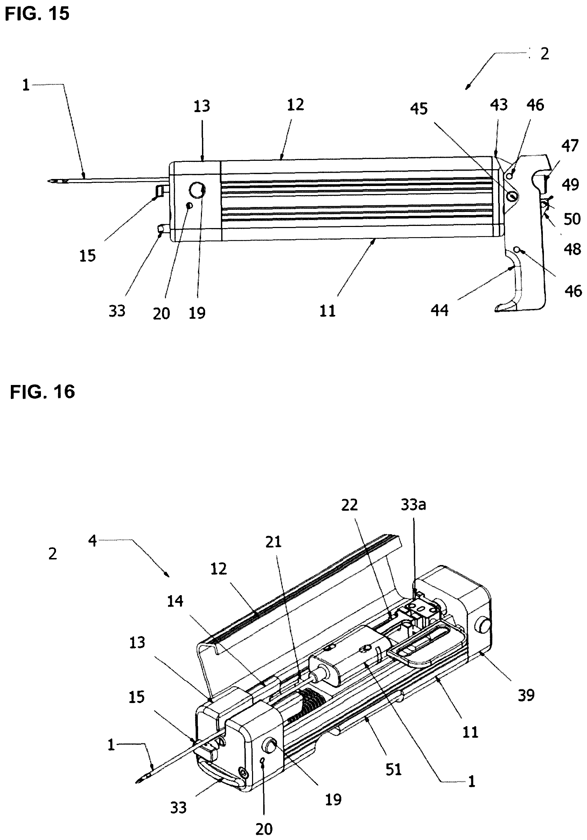

Figure 15 illustrates a side view of the trigger with the lid closed and needle coupled, of the option of model with handle of the lever type;
Figure 16 shows a perspective upper-front view of the trigger with needle coupled, of the option of model with lower handle with cross recess, a compartment to fit the user's fingers;

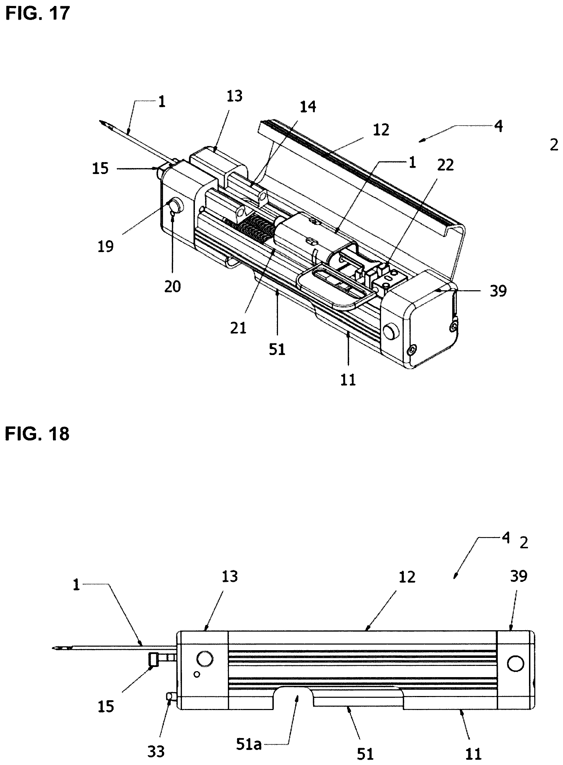

Figure 17 reveals a perspective upper-back view of the trigger with needle coupled, of the option of model with lower handle with cross recess, a compartment to fit the user's fingers;
Figure 18 illustrates a side view of the trigger with needle coupled, of the option of model with lower handle with cross recess, where the compartment to fit the user's fingers is visualized better.

### DETAILED DESCRIPTION OF THE INVENTION

With a reference to the illustrated drawings, the present patent of utility model refers to **"DISPOSITION APPLIED TO A DEVICE FOR BIOPSY WITH REUSABLE TRIGGER FOR DISPOSABLE NEEDLE",** more precisely, it is equipment for histological biopsy, composed of a set of disposable needles and a reusable trigger; the needles were designed and developed for exclusive use with such trigger, and it is not possible to use them in other equipment.

Constituted from a structural box (11) in horizontal rectangular prismatic shape, in whose front portion there is a front compartment, with pivot opening lid (12), in which front carrier (21) and back carrier (22) are installed, aligned in series and fitted in guide rails (11a), designed on the side walls of the front compartment, front carrier (21) has rectangular prismatic shape, in whose side faces, there are rectangular cams (21c), which are fitted in guide rails (11a), in the lower face of the front side of front carrier (21), there is a cavity to seat helical springs (41), which provides the propulsion of the set of front (21) and back (22) carriers, which are engaged by means of a cross cylindrical shaft (20) on front lid (13) and another cross cylindrical shaft (21b) located on front carrier (21). On the upper face of front carrier (21), there are two pins (21a), placed in diagonal, where the main cannon (2) is fitted.

The main cannon (2) has rectangular shape, with the side and the front edges side rounded and a circular cam to the center of the front part. A rotation movement is generated at the beginning and at the end of the longitudinal movement caused by the activation of the rotation latch (8) coupled to cannon main (2), which is driven by back carrier (22), which is projected until touching front carrier (21), which move after the release of helical springs (41) fixed in front carrier (21), which is interconnected to back carrier (22) by a cylindrical shaft (25) in the longitudinal direction. The rotation latch (8) slides through the threaded cannon (6) generating rotation movement around the longitudinal shaft. Cannula (6a) and cannula (7a) are placed coaxially to the mandrel (9a) with a pyramidal tip and rectangular cross recess close to the pyramidal tip to relieve the internal pressure, it is placed on the support of the mandrel (9) and it is coupled to the base (31) by means of cylindrical pins (31a) located on the front face of the back lid (39), which is fixed to structural box (11) by means of cylindrical-headed screws (39a). In internal cannula (7a), there are two oblique cavities close to the front part, opposite in the cross direction, however distanced in the longitudinal direction, which enable introduction of the latches of external cannula (6a), when helical springs (3) are compressed after cannon main (2) touches the forward limit (14) displacing the support of threaded cannon (4). Internal cannula (7a) enables making different types of bevels, according to its application, as demonstrated on Figure (3).

On the upper face of back carrier (22), there is a prismatic rectangular projection in the cross direction, a channel to the center of the flat base and a channel with circular bases in the longitudinal direction, in front lid (13), there is cross circular shaft (20) where the helical springs (41) are engaged. Front lid (13) is provided with a back compartment, where forward limit (14) is seated, which is interconnected to front lid (13) by cylindrical shafts (17) with bigger diameter in the back part, which protects helical springs (17a).

Forward control button (15) is placed on the front face of the forward limit (14) by means of cylindrical shaft with recess in the lower front part (15a). Locking button (16) of forward limit (14) is located on the left side face of front lid (13), which, after forward control button (15) is activated for positioning to collect smaller size, is responsible to release central limit (14) to the initial position. Activation button (19) of lid closing lock (18) is located on the right side face of front lid (13) and lid closing lock (18) in the back part of front lid (13).

Back lid (39) is provided with a cavity at the left side, in rectangular shape with rounded edges, to seat the side trigger (35) with a circular channel to the center, on the right side, there is safety lock button (37) with cross rod to the front face, which intercepts the locking shaft (38), in cylindrical shape with oblique detail in the 1^{st} quadrant, locking shaft (38) is fixed to cylindrical pin (38b) in vertical position, which interconnects activation rod (38c). In the lower base of the lid (39) is fixed the support (40) in prismatic shape with rounded edges, to help in the equipment handling.

The trigger is placed in the triggering position by means of handle (23), in prismatic shape and rounded lower edges, in whose lower face, there is a cavity to seat and fix dragging handle (23a), in rectangular shape with rounded edges and anatomic shape in the back part. In its upper face, handle (23) is provided with a projection in the shape of a triangle to unlock levers (27), which are moved forward in parallel by spring (28), whose function is to keep back carrier (22) at the correct distance.

Some observations shall be highlighted, such as: handle (44) in the back part of back lid (43) placed in the form of a lever can also be used to manufacture the trigger equipment for biopsy (2); its shape is rectangular with cavity passing along its vertical extension, whose cavity enables displacement of levers (50) inside, which move central shaft (49), which drives the entire internal mechanism by traction to set position, where it is locked by lever (32), the system is provided with safety lock (45) in the back part of the back lid.

Trigger equipment (2) can also be provided with lower handle (51), which moves through a cross opening in structural box (11) which, on its turn, is provided with cavity that enables fitting the user's fingers to drive the internal mechanisms to set position, where it is locked by lever (32).

It is certain that, when the present invention is placed in practice, there might be modifications regarding given construction and shape details, but this will not affect the fundamental principles, which are clearly grounded in the claim section, thus, it is understood that the used terminology is not limited.

Thus, the present description report is an innovating concept, which presents constructive and functional characteristics as it can be evidenced by the analyses carried out and the figures shown.

By the advantages it offers, and as its characteristics are truly innovating and fulfill all requirements of innovation and originality in the art, the present **"DISPOSITION APPLIED TO A DEVICE FOR BIOPSY WITH REUSABLE TRIGGER FOR DISPOSABLE NEEDLE"** gathers the conditions necessary to deserve the patent of right of invention.

## Claims

1. **"DISPOSITION APPLIED TO A DEVICE FOR BIOPSY WITH REUSABLE TRIGGER FOR DISPOSABLE NEEDLE"** refers to equipment for histological biopsy, **characterized for** being provided with reusable trigger (2) with single activation to set and double stage to collect samples of soft tissues for biopsy; constituted from a structural box (11) in horizontal rectangular prismatic shape, in whose front portion there is a compartment with pivoting opening lid (12), in which front carrier (21) and back carrier (22) are installed, aligned in series and fitted in guide rails (11a) designed on the side walls of the front compartment, front carrier (21) has a rectangular prismatic shape, in whose side faces there are rectangular cams (21c), which are fitted in the guide rails (11a), considering that carrier (21) is interconnected to back carrier (22) by cylindrical shaft (25), which crosses a tunnel in the longitudinal direction of carrier (21) with scaled diameter, which protects helical spring (26) that drive back carrier (22), which has a longitudinal hole with threaded circular section for the fixation of cylindrical shaft (25); front carrier (21) is provided with a cavity for seating of helical springs (41) which, upon setting trigger (2), store the potential elastic power to provide the propulsion of the internal mechanism, whose main items are carriers (21) and (22) and needle (1), which is coupled on the upper face of front carrier (21), which is provided with two pins placed in diagonal, where main cannon (2) is fitted; helical springs (41) drive the mechanism against front lid (13) performing the necessary trip for the biopsy procedure until front carrier (21) touches its front face on forward limit (14); front lid (13) is provided with cross circular shaft (20), where helical springs (41) are engaged; front lid (13) is provided with a back compartment, where forward limit (14) is seated, which is interconnected to front lid (13) by circular shafts (17) with bigger diameter in the back part, which protects helical springs (17a), forward control button (15) is located on the front face of forward limit (14) by means of the cylindrical shaft with recess in the lower front part (15a); said limit is responsible for the size of the fragment taken in biopsy, to return to the previous position, there is lock button (16) which is located on the left side face of front lid (13); activation button (19) is responsible for the release of the closing lock of lid (18) and it is located on the right side face of front lid (13) and the closing lock of lid (18) in the back part of front lid (13); back lid (39) is provided with a cavity on the left side, in rectangular shape with rounded edges, for seating of side catch (35) with a circular channel to the center, in the right side, there is safety lock button (37) with cross rod to the front face, which intercepts locking shaft (38) in cylindrical shape with oblique detail in the 1^{st} quadrant, locking shaft (38) is provided with cylindrical pin (38b) at the vertical, which interconnects driving rod (38c); at the lower base of lid (39), there is support (40) in prismatic shape with rounded edges to help the equipment handling; the trigger is set for triggering by means of handle (23) in prismatic shape with rounded lower edges, in whose lower face, there is a cavity for seating and fixation of dragging handle (23a) in rectangular shape with rounded edges and anatomic shape in the back part; in the upper face of handle (23), there is a projection in the shape of a triangle to unlock levers (27) for the distancing of back carrier (22), upon unlocking levers (27), rotation movement is generated at the beginning and at the end of the longitudinal movement of needle (1), due to the activation of rotation latch (8) coupled to main cannon (2), which is driven by back carrier (22), which is projected until touching front carrier (21), which moves after the release of helical springs (41) fixed on front carrier (21); rotation latch (8) slides along threaded cannon (6), generating rotation movement around the longitudinal shaft.

2. **"DISPOSITION APPLIED TO A DEVICE FOR BIOPSY WITH REUSABLE TRIGGER FOR DISPOSABLE NEEDLE",** accordingtoclaim number one, **characterized for** dispose of a disposable needle, exclusively manufactured to be used in the reusable trigger mentioned in the present petition; the needle is provided with a longitudinal displacement system of the set of cannulas (internal and external) and their simultaneous rotation movement, considering that such rotation movement occurs first at the beginning of the forward movement of the cannulas, cutting the tissue with the purpose to facilitate their penetration; the cannulas (6a and 7a) are placed coaxially towards the mandrel (9a) with pyramidal tip and rectangular cross recess, close to the pyramidal tip to relieve the internal pressure, mandrel (9a) is placed in mandrel support (9) on the front face of back lid (39), which is fixed on structural box (11); internal cannula (7a) is provided with two opposed oblique cavities, close to the front part, which enables the introduction of the latches of external cannula (6a) when helical springs (3) are compressed after main cannon (2) touches forward limit (14) displacing the support of threaded cannon (4); internal cannula (7a) enables different types of bevels to be made according to its deployment, as demonstrated on Figure 3; the triggers for release of springs (41) can be made after pressing side trigger button (35) in rectangular shape with rounded edges or front trigger (33) in cylindrical shape with a U-shaped bend with rectified surface.
